(19) Europäisches Patentamt
European Patent Office
Office européen des brevets

(11) EP 1 186 673 A2

(12) **EUROPEAN PATENT APPLICATION**

(43) Date of publication:
**13.03.2002 Bulletin 2002/11**

(51) Int Cl.[7]: **C12Q 1/68**

(21) Application number: **01307665.8**

(22) Date of filing: **10.09.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **11.09.2000 US 659173**

(71) Applicant: **Agilent Technologies Inc**
**Palo Alto, CA 94306-2024 (US)**

(72) Inventors:
- **Wobler, Paul K.**
  **Los Altos, California 94022 (US)**
- **Delenstarr, Glenda C.**
  **Belmont, California 94002 (US)**

(74) Representative: **Tollett, Ian et al**
**Williams, Powell & Associates,**
**4 St. Paul's Churchyard Inn**
**London EC4M 8AY (GB)**

(54) **Calibration of molecular array data**

(57) A method for calibrating different types of signals scanned from a molecular array (900), or calibrating signals scanned from different molecular arrays, by employing calibrating probes that generate signals proportional to the total concentrations of labeled target molecules to which the molecular array probes are directed over an entire range of sample solutions, and molecular arrays incorporating sets of calibrating probes. For molecular arrays that include oligonucleotide probes directed to cDNA targets produced by reverse transcription of mRNA molecules, suitable probes for calibrating features include: (1) poly(A) oligonucleotides of varying lengths; (2) oligonucleotides having sequences complementary to cDNA copies of cDNA transcripts of Alu repeat sequences in human mRNA molecules; (3) oligonucleotide probes complementary to arbitrary synthetic sequences incorporated into 5'-end primers used to initiate reverse transcription of mRNA molecules; and (4) random oligonucleotide probes of varying lengths with high probability of being complementary to relatively large fractions of target molecules.

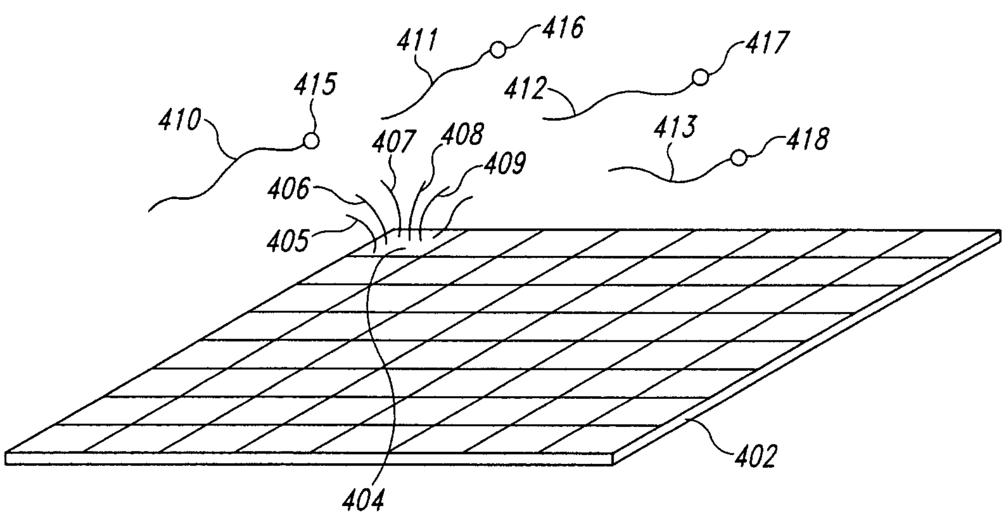


Fig. 4

EP 1 186 673 A2

## Description

The present invention relates to methodologies for processing raw data generated from experiments based on molecular arrays, and, in particular, to a method for calibrating signal data from molecular arrays or, in other words, for determining the correspondence between signals read from features of a molecular array by optical scanning or radiometric scanning and the concentrations of labeled target molecules present in a sample solution to which the molecular array was exposed. The invention also relates to a molecular array.

The present invention is related to molecular-array-based analysis of complex solutions, including applications involving analysis of complex solutions containing many different types of intermediate-length nucleic acid polymers along with other types of biopolymers and organic and inorganic molecules. In these applications, the goal of molecular-array-based analysis is to determine the concentrations of particular nucleic-acid polymers in complex sample solutions. Molecular-array-based analytical techniques are not, however, restricted to analysis of nucleic acid solutions, but may be employed to analyze complex solutions of any type of molecule that can be optically or radiometrically scanned and that can bind with high specificity to complementary molecules synthesized within, or bound to, discrete features on the surface of a molecular array. Because molecular arrays are widely used for analysis of nucleic acid samples, the following background information on molecular arrays will be introduced in the context of analysis of nucleic acid solutions, particularly deoxyribonucleic acid ("DNA") solutions, following a brief background description of nucleic acid chemistry. However, RNA solutions, synthetic nucleotide polymer solutions, and other types of sample solutions may have alternatively been chosen for the following illustrations.

DNA and ribonucleic acid ("RNA") are linear polymers, each synthesized from four different types of subunit molecules. The subunit molecules for DNA include: (1) deoxy-adenosine, abbreviated "A," a purine nucleoside; (2) deoxy-thymidine, abbreviated "T," a pyrimidine nucleoside; (3) deoxy-cytosine, abbreviated "C," a pyrimidine nucleoside; and (4) deoxy-guanosine, abbreviated "G," a purine nucleoside. The subunit molecules for RNA include: (1) adenosine, abbreviated "A," a purine nucleoside; (2) uracil, abbreviated "U," a pyrimidine nucleoside; (3) cytosine, abbreviated "C," a pyrimidine nucleoside; and (4) guanosine, abbreviated "G," a purine nucleoside. Figure 1 illustrates a short DNA polymer 100, called an oligomer, composed of the following subunits: (1) deoxy-adenosine 102; (2) deoxy-thymidine 104; (3) deoxy-cytosine 106; and (4) deoxy-guanosine 108. When phosphorylated, subunits of DNA and RNA molecules are called "nucleotides" and are linked together through phosphodiester bonds 110-115 to form DNA and RNA polymers. A linear DNA molecule, such as the oligomer shown in Figure 1, has a 5' end 118 and a 3' end 120. A DNA polymer can be chemically characterized by writing, in sequence from the 5' end to the 3' end, the single letter abbreviations for the nucleotide subunits that together compose the DNA polymer. For example, the oligomer 100 shown in Figure 1 can be chemically represented as "ATCG." A DNA nucleotide comprises a purine or pyrimidine base (e.g. adenine 122 of the deoxy-adenylate nucleotide 102), a deoxy-ribose sugar (e.g. deoxy-ribose 124 of the deoxy-adenylate nucleotide 102), and a phosphate group (e.g. phosphate 126) that links one nucleotide to another nucleotide in the DNA polymer. In RNA polymers, the nucleotides contain ribose sugars rather than deoxy-ribose sugars. In ribose, a hydroxyl group takes the place of the 2' hydrogen 128 in a DNA nucleotide. RNA polymers contain uridine nucleosides rather than the deoxy-thymidine nucleosides contained in DNA. The pyrimidine base uracil lacks a methyl group (130 in Figure 1) contained in the pyrimidine base thymine of deoxy-thymidine.

The DNA polymers that contain the organization information for living organisms occur in the nuclei of cells in pairs, forming double-stranded DNA helixes. One polymer of the pair is laid out in a 5' to 3' direction, and the other polymer of the pair is laid out in a 3' to 5' direction. The two DNA polymers in a double-stranded DNA helix are therefore described as being anti-parallel. The two DNA polymers, or strands, within a double-stranded DNA helix are bound to each other through attractive forces including hydrophobic interactions between stacked purine and pyrimidine bases and hydrogen bonding between purine and pyrimidine bases, the attractive forces emphasized by conformational constraints of DNA polymers. Because of a number of chemical and topographic constraints, double-stranded DNA helices are most stable when deoxy-adenylate subunits of one strand hydrogen bond to deoxy-thymidylate subunits of the other strand, and deoxy-guanylate subunits of one strand hydrogen bond to corresponding deoxy-cytidilate subunits of the other strand.

Figures 2A-B illustrate the hydrogen bonding between the purine and pyrimidine bases of two anti-parallel DNA strands. Figure 2A shows hydrogen bonding between adenine and thymine bases of corresponding adenosine and thymidine subunits, and Figure 2B shows hydrogen bonding between guanine and cytosine bases of corresponding guanosine and cytosine subunits. Note that there are two hydrogen bonds 202 and 203 in the adenine/thymine base pair, and three hydrogen bonds 204-206 in the guanosine/cytosine base pair, as a result of which GC base pairs contribute greater thermodynamic stability to DNA duplexes than AT base pairs. AT and GC base pairs, illustrated in Figures 2A-B, are known as Watson-Crick ("WC") base pairs.

Two DNA strands linked together by hydrogen bonds forms the familiar helix structure of a double-stranded DNA helix. Figure 3 illustrates a short section of a DNA double helix 300 comprising a first strand 302 and a second, anti-parallel strand 304. The ribbon-like strands in Figure 3 represent the deoxyribose and phosphate backbones of the two anti-parallel strands, with hydrogen-bonding purine and pyrimidine base pairs, such as base pair 306, interconnecting the two strands. Deoxy-guanylate subunits of one strand are generally paired with deoxy-cytidilate subunits from the other strand, and deoxy-cytidilate subunits in one strand are generally paired with deoxy-adenylate subunits from the other strand. However, non-WC base pairings may occur within double-stranded DNA. Generally, purine/pyrimidine non-WC base pairings contribute little to the thermodynamic stability of a DNA duplex, but generally do not destabilize a duplex otherwise stabilized by WC base pairs. However, purine/purine base pairs may destabilize DNA duplexes.

Double-stranded DNA may be denatured, or converted into single stranded DNA, by changing the ionic strength of the solution containing the double-stranded DNA or by raising the temperature of the solution. Single-stranded DNA polymers may be renatured, or converted back into DNA duplexes, by reversing the denaturing conditions, for example by lowering the temperature of the solution containing complementary single-stranded DNA polymers. During renaturing or hybridization, complementary bases of anti-parallel DNA strands form WC base pairs in a cooperative fashion, leading to regions of DNA duplex. Strictly A-T and G-C complementarity between anti-parallel polymers leads to the greatest thermodynamic stability, but partial complementarity including non-WC base pairing may also occur to produce relatively stable associations between partially-complementary polymers. In general, the longer the regions of consecutive WC base pairing between two nucleic acid polymers, the greater the stability of hybridization between the two polymers under renaturing conditions.

The ability to denature and renature double-stranded DNA has led to development of many extremely powerful and discriminating assay technologies for identifying the presence of DNA and RNA polymers having particular base sequences or containing particular base subsequences within complex mixtures of different nucleic acid polymers, other biopolymers, and inorganic and organic chemical compounds. These methodologies include molecular-array-based hybridization assays. Figures 4-7 illustrate the principle of molecular-array-based hybridization assays. A molecular array (402 in Figure 4) comprises a substrate upon which a regular pattern of features is prepared by various different types of manufacturing processes. The molecular array 402 in Figure 4, and in subsequent Figures 5-7, has a grid-like two-dimensional array of regularly shaped features, such as feature 404 shown in the upper left-hand corner of the molecular array. Each feature of the molecular array contains a large number of identical oligonucleotides covalently bound to the surface of the feature. In general, chemically distinct oligonucleotides are bound to the different features of a molecular array, so that each feature corresponds to a particular nucleotide sequence. In Figures 4-6, the principle of molecular-array-based hybridization assays is illustrated with respect to the single feature 404 to which a number of identical oligonucleotides 405-409 are bound. In practice, each feature of the molecular array contains an enormous number of oligonucleotide molecules, but, for the sake of clarity, Figures 4-6 only show a small number.

Once a molecular array has been prepared, the molecular array may be exposed to a sample solution of DNA molecules that includes DNA molecules (410-413 in Figure 4) labeled with fluorophores, chemoluminescent compounds, or radioactive atoms 415-418. A labeled DNA molecule that contains a nucleotide sequence complementary to the base sequence of an oligonucleotide bound to the molecular array may hybridize through base pairing interactions to the oligonucleotide. Figure 5 shows a number of labeled DNA molecules 502-504 hybridized to oligonucleotides 505-507 bound to the surface of the molecular array 402. DNA molecules that do not contain nucleotide sequences complementary to any of the oligonucleotides bound to the molecular array do not hybridize stably to oligonucleotides bound to the molecular array and generally remain in solution, such as labeled DNA molecules 508 and 509. The sample solution is then rinsed from the surface of the molecular array, washing away any unbound labeled DNA molecules. Finally, as shown in Figure 6, the bound labeled DNA molecules are detected via optical or radiometric scanning. Optical scanning involves exciting labels of bound labeled DNA molecules with electromagnetic radiation of appropriate frequency and detecting fluorescent emissions from the labels, or detecting light emitted from chemoluminescent labels. When radioisotope labels are employed, radiometric scanning can be used to detect radiation emitted from labeled DNA molecules hybridized to oligonucleotides bound to the surface of the molecular array. Optical or radiometric scanning produces an analog or digital representation of the molecular array as shown in Figure 7, with features to which labeled DNA molecules are hybridized similar to 706 optically or digitally differentiated from those features to which no labeled DNA molecules are bound. In other words, the analog or digital representation of a scanned molecular array displays positive signals for features to which labeled DNA molecules are hybridized and displays signals indistinguishable from the measurement background for features to which no labeled DNA molecules are bound. Features displaying positive signals in the analog or digital representation indicate the presence of DNA molecules with complementary nucleotide sequences in the original sample solution. Moreover, the signal intensity produced by a feature is generally related to the amount of labeled DNA bound to the feature, which is in turn related to the concentration, in the sample to which the molecular array was exposed, of labeled DNA complementary to the oligonucleotide within the feature.

Molecular-array-based hybridization techniques allow extremely complex solutions of DNA molecules to be analyzed in a single experiment. Molecular arrays may contain hundreds, thousands, or tens of thousands or different oligonucleotides, allowing for the detection of hundreds, thousands, or tens of thousands of different DNA polymers containing complementary nucleotide subsequences in the complex DNA solutions to which the molecular array is exposed. In order to perform different sets of hybridization analyses, molecular arrays containing different sets of bound oligonucleotides are manufactured by any of a number of complex manufacturing techniques. These techniques generally involve synthesizing the oligonucleotides within corresponding features of the molecular array through complex iterative synthetic steps.

As pointed out above, molecular-array-based assays can involve other types of biopolymers. For example, one might attach protein antibodies to features of the molecular array that would bind to soluble labeled antigens in a sample solution. Many other types of chemical assays may be facilitated by molecular array technologies.

The calibration problem, to which the present invention is related, is illustrated with reference to Figures 8A-C in a simple, abstract, hypothetical example of a gene expression experiment. The intent of the experiment is to detect which of genes *p, q, r*, and *s* are up-regulated in response to exposure of an organism to a pharmaceutical agent, and thus produce greater concentrations of their respective mRNA transcription products, and which of genes *p, q, r*, and *s* are down-regulated in response to exposure of the organism to the pharmaceutical agent, and produce lower concentrations of their respective mRNA transcription products.

Figure 8A shows a simple four-feature molecular array 800 in which feature 1 801 contains bound oligonucleotides with a sequence represented by the letter "P," such as bound oligonucleotide 802, and features 2-4 (803-805, respectively) contain oligonucleotides with sequences represented by the letters "Q," "R," and "S," respectively. Sequences "P," "Q," "R," and "S," can be considered to be unique subsequences of or complements to subsequence genes *p*, or complements to subsequences of, genes *p, q, r*, and *s*, respectively. The oligonucleotides P-S, covalently bound to features of the molecular array 800, are referred to as "probes."

In Figure 8B, the four-feature molecular array 800 is exposed to a sample solution 810 containing various labeled cDNA transcripts of messenger RNA ("mRNA") molecules. This sample solution may be prepared from a first solution of mRNA molecules purified from a cell extract solution obtained from an organism prior to exposure of the organism to a particular pharmaceutical agent and from a second solution of mRNA molecules purified from a cell extract solution obtained from the organism following exposure of the organism to the pharmaceutical agent. The mRNA molecules are the products of gene expression, transcribed from genes by an RNA polymerase. The first and second solutions of mRNA molecules may be incubated with reverse transcriptase, deoxy-nucleotide-triphosphates, and two different labeled deoxynucleotide triphosphate analogues to generate two different types of cDNA molecules complementary to the mRNA molecules. The first sample solution, for example, may be incubated with a first, red-chromophore-labeled triphosphate analogue, and the second sample solution may be incubated with a second, green-chromophore-labeled triphosphate analogue. Thus, red-chromophore-labeled cDNA molecules are derived from the first solution, obtained from the cell extract solution of the organism prior to exposure of the organism to the pharmaceutical agent, and the green-chromophore-labeled cDNA molecules are derived from the second solution, obtained from the cell extract solution of the organism following exposure of the organism to the pharmaceutical agent. The sample solution 810, prepared by mixing the red-chromophore-labeled and green-chromophore-labeled cDNA solutions, includes labeled cDNA molecules with sequences "P'," "Q'," "R'," and "S'" complementary to the probe sequences P, Q, R, and S, respectively. In Figure 8B, red-chromophore-labeled molecules are indicated with unfilled disks at one end, and green-chromophore-labeled molecules are indicated with filled disks at one end, the other ends of the molecules having an indication of the sequence of the molecule, such as the sequences "P'," "Q'," "R'," and "S'."

By incorporating probes molecules with sequences P, Q, R, and S, the molecular array 800 has been designed to detect the presence of cDNA copies of the cDNA transcripts of the four mRNA transcripts of genes *p, q, r*, and *s*. The cDNA complementary to the oligonucleotide probe bound to a particular feature is called the "target" cDNA molecule for that feature or for that probe. In the sample solution, some cDNA molecules are labeled with a chromophore that produces a red wavelength signal when illuminated during scanning, indicated in Figure 8B by unfilled circles, such as unfilled circle 811, at one end of the abstract representations of the cDNA molecules. Label molecules or atoms can be incorporated into target molecules during synthesis of the target molecules by employing labeled monomer substrates, and by other means known in the art. Alternatively, chrornophores and radiolabels may be added after hybridization to bind covalently or non-covalently to specific chemical moieties, sites, or subsequences within target molecules. Note also that both sense and antisense probes may be employed in molecular arrays.

After the target cDNA molecules in the sample solution 810 having sequences P', Q', R', and S' are allowed to hybridize, under renaturing conditions, to probe oligonucleotides with complementary sequences bound to the molecular array, the sample solution is rinsed from the surface of the molecular array to leave target cDNA molecules labeled with red and green chromophores bound to complementary oligonucleotide probes on the surface of the molecular array. Figure 8C illustrates target cDNA molecules with red and green chromophore labels bound to complementary oligonucleotide probes on the surface of the molecular array.

At this point, the molecular array can be analyzed by optical scanning techniques to determine the intensity of red and green light emitted by the red and green chromophores bound to target cDNA molecules hybridized to probe oligonucleotides on the surface of the molecular array. Scanning of the molecular array for red light emitted by the red chromophores produces a set of red signals with a range of different red signal intensities possible for each feature scanned, and scanning of the molecular array for green light emitted by the green chromophores produces a set of green signals with a range of different green signal intensities possible for each feature scanned. For a given feature, the ratio of the measured green signal intensity to the measured red signal intensity is related to the ratio of the concentration of that feature's target cDNA in the second sample solution to the concentration of that feature's target cDNA in the first sample solution. If the measured green and red signals are directly related to concentrations of red-chromophore-labeled and green-chromophore-labeled cDNA molecules in their respective sample solutions, then the ratio of green signal to red signal for a feature directly indicates the degree to which the corresponding gene is over-regulated or under-regulated following exposure of the organism to the pharmaceutical agent

For example, Table 1, below, shows hypothetical concentrations of each of the labeled cDNA copies of mRNA transcripts of hypothetical genes *p, q, r,* and *s* of the sample solution of Figures 8B, along with the ratios of the concentrations:

| | *p* | *q* | *r* | *s* |
|---|---|---|---|---|
| $\circ_c$ | 1 | 200 | 7 | 1 |
| $\bullet_c$ | 5 | 400 | 2 | 1 |
| $\bullet_c / \circ_c$ | 5 | 2 | 0.286 | 1 |

**Table 1**

In this and the following tables and figures, unfilled subscripted circles represent red and filled subscripted circles represent green, with the subscripts "c," "o," and "n" indicating "concentration." "observed," and "normal," respectively. Thus, " $\circ_c$ " represents the concentration of red-chromophore-labeled cDNA, " $\circ_o$ " represents the red signal scanned from one or more features of a molecular array, and " $\circ_n$ " represents a normalized value for the red signal scanned from one or more features of a molecular array. The concentrations in Table 1 are given as integers corresponding to some arbitrary unit of measurement.

Table 1 includes, in the last row, the green-signal-to-red-signal ratios or, equivalently, the green-chromophore-labeled target concentration to red-chromophore-labeled target concentration ratios for the four target cDNA copies of the mRNA molecules expressed from genes *p*, *q, r*, and *s*. The green-signal-to-red-signal ratio for cDNA copies of the mRNA expressed from the *s* gene is equal to "1," indicating that expression of gene *s* does not change in response to exposure of the organism to the pharmaceutical agent. The green-to-red-signal ratios measured for the features corresponding to the mRNA expressed from genes *p* and *q* are significantly higher than one, indicating that genes *p* and *q* are more actively transcribed in the organism following exposure of the organism to the pharmaceutical agent. The green-signal-to-red-signal ratio for the target cDNA copy of the mRNA expressed from gene *r* is significantly lower than one, indicating that gene *r* is expressed at a lower level in the organism following exposure to the pharmaceutical agent. In typical gene expression experiments, the molecular array may contain thousands or hundreds of thousands of different features, each containing a probe oligonucleotide complementary to a different labeled cDNA target molecule, so that the gene expression levels of thousands or hundreds of thousands of genes can be determined for an organism at discrete points in time in order to monitor overall gene expression within the organism over a period of time.

The simple direct relationship between signal intensity and sample concentration is generally not experimentally observed. First, for many different reasons, the amount of chromophore-labeled target molecules that hybridize to probe molecules on the surface of a molecular array following an experiment may not be directly proportional to the concentration of the target molecules in the sample solution to which the molecular array was exposed. For example, the kinetic and thermodynamic properties of the probe and target molecules will cause some binding reactions to occur much more efficiently than others. This effect is illustrated in Table 2, below, where the binding efficiency of a target and its complementary probe is assumed to be the same for both the red-chromophore-labeled and the green-chromophore-labeled versions of the target, the binding efficiencies $E_p$, $E_q$, $E_r$, $E_s$ for the target cDNA copies of mRNA transcripts of genes *p, q, r,* and s are 0.5, 0.9, 0.1, and 0.7, respectively, and the effective surface concentrations or densities of the labeled target molecules bound to their respective probe molecules on the surface of the molecular array are $Ceffective_i = E_i * [target_i]$:

| | *p* | *q* | *r* | *s* |
|---|---|---|---|---|
| 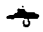 | 0.5 | 180 | 0.7 | 0.7 |
| 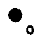 | 2.5 | 360 | 0.2 | 0.7 |
| 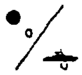 | 5 | 2 | 0.286 | 1 |

**Table 2**

As can be seen from Table 2, the ratios calculated from the observed red and green signals included in the first two rows of Table 2 are the same as those included in the last row of Table 1, demonstrating that the effects of differing binding efficiencies cancel upon calculation of the green-to-red-signal ratios. A second phenomenon that contributes to the lack of proportionality between measured signal intensities and absolute solution concentrations of target molecules is that different chromophores may absorb and emit different amounts of light on a per molecule basis. Similarly, optical detectors may be more sensitive, or produce stronger signals, in response to certain wavelengths of light. In addition, targets may interact with the surface and with each other in a concentration-dependent manner. Thus, for example, in the current hypothetical case, the measured green signal intensities may be roughly proportional to twenty times the surface densities or surface concentrations of green chromophores, shown in Table 2, while the measured intensities from red chromophores may be thirty times the surface densities or surface concentrations shown in Table 2 raised to the power "1.1." Stated more concisely:

$$S_{Gi} = 20(E_i * [target_i]) = 20 * Ceffective_i$$

$$S_{Ri} = 30(E_i * [target_i])^{1.1} = 30 * (Ceffective_i)^{1.1}$$

In the current hypothetical case, the measured intensities of the green and red signals, according to above-described formulas relating measured red and green signal intensities to sample concentrations and binding efficiencies, are shown in Table 3:

| | p | q | r | s |
|---|---|---|---|---|
| 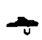 | 14 | 9076 | 20.2 | 20.2 |
| 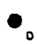 | 50 | 7200 | 4 | 14 |
| 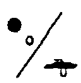 | 3.45 | 0.77 | 0.19 | 0.67 |

**Table 3**

Note that the data in Table 3 include both the effects of non-proportionality between solution concentrations of target molecules and the resulting densities of hybridized target molecules on the surface of the molecular array as well as the different efficiencies of signal production by green and red chiomophores and signal detection by optical instrumentation. Note further that the operation of calculating ratios does not compensate for these effects, i.e. the ratios calculated from Table 3 are not the same as those calculated from Tables 1 and 2. Because of the various non-proportionalities described above, but principally because the lack of normalization between the green signal data and the red signal data, over-expression of gene *p* is now underestimated, genes *q* and *s* appear to be repressed following exposure of the organism to the pharmaceutical agent, and expression of gene r appears to be much more repressed than it actually was, based on the absolute solution concentrations shown in Table 1.

The above discussion, with reference to Figures 8A-C and Tables 1-3, illustrates that raw signal ratio data derived from optical scanning of molecular arrays cannot be directly used to determine relative levels of gene expression from one set of signal intensities to another. In practice, many additional complicating factors may be present. For example, the discrepancies between the efficiencies of chromophores and the efficiency of detecting signals from chromophores may not be linear with respect to the density of chromophores bound to the surface of a molecular array, but may be proportional to some non-linear function of density. Many other factors may contnbute to a lack of proportionality between the density of hybridized target molecules bound to different features of a molecular array and the corresponding concentrations of the target molecules of the features in sample solutions. The simple example illustrated in Figures 8A-C relates to discrepancies between measured red and green signals, but sirnilar discrepancies can arise between signals of one type measured from different molecular arrays. One of the primary goals of initial data processing carried out on data sets obtained by scanning molecular arrays is to normalize different data sets with respect to one another in order to account for differences in the efficiencies of signal production by different types of labels, differences between different molecular arrays, and differences in efficiencies by which different types of signals are measured by scanning instrumentation. In the above example, normalization of two data sets corresponding to two different types of signals is considered, but normalization techniques need also to be applied to normalize more than two data sets corresponding to more than two signals generated during experiments that employ numerous types of signal-producing labels.

Experimeritalists and designers and manufacturers of molecular arrays and molecular array data processing systems have thus recognized a need for a simple, reliable, and efficient method and system for calibrating data generated from analysis of molecular arrays, so that, for example, gene expression levels can generated from observed relative signal intensities.

The present invention is directed towards calibrating signals scanned from the features of a molecular array to concentrations of target molecules for those features present in a sample solution to which the molecular array has been exposed. Signals corresponding to different labels bound to the features of a molecular array, or signals corresponding to a single label bound to of two or more molecular arrays, may not be proportional to the relative concentrations of target molecules in sample solutions to which probe molecules bound to the features of a molecular array are directed. The lack of proportionality may arise because of varying intensities of light emitted by chromophore labels or radiation emitted by radioactive labels and because of varying responses of scanning instrumentation to signals produced by different labels. The lack of proportionality may also arise for particular features because of interactions of the target molecules to which the features are directed and other molecules in sample solutions, from defects in the deposition or synthesis of probe molecules on the surface of the molecular array, and other chemically related phenomena. The former signal response problems are generally constant for a given label and instrumental scanning technique. The latter problems related to the unforeseen chemical interactions between target molecules, unforeseen interactions between sample molecules and particular probes, and other such chemical phenomena, tend to be highly dependent on the specific chemical identity of particular target and probe molecules as well as on the type of sample solution containing the target molecules.

According to one embodiment of the present invention, a set of calibrating probes is chosen to generate signals proportional to the total concentrations of labeled target molecules to which the calibration probes are directed over the entire range of sample solutions to which a molecular array is experimentally exposed is chosen. If error sources are approximately linear, signals produced from each feature in the molecular array are normalized to the average signal generated by the calibrating features. If some or all of the error sources are non-linear, signals produced from each feature in the molecular array are normalized to a system response function determined from the signal generated by the calibrating features. A correspondence between the signal generated by each feature and the mole fraction in the sample solution of the target molecule to which the feature is directed can then be determined. For molecular arrays that include oligonucleotide probes directed to cDNA produced by reverse transcription of mRNA molecules or cRNA produced by reverse transcription of mRNA molecules followed by *in vitro* transcription of RNA, commonly used to determine the levels of gene expression in different tissues or at different points in time, suitable probes for calibrating features include: (1) poly(A) oligonucleotides of varying lengths complementary to 3' poly(T) tails of cDNA copies of cDNA transcripts of eukaryotic mRNA molecules; (2) poly(A)-containing oligonucleotides of varying lengths complementary to 3' poly('I)-containing tails of cRNA copies of cDNA transcripts of eukaryotic mRNA molecules; (3) oligonucleotides having sequences complementary to cDNA copies of cDNA transcripts of Alu repeat sequences that commonly occur in human mRNA molecules; (4) oligonucleotide probes complementary to arbitrary synthetic sequences incorporated into the 5'-end primers used to initiate reverse transcription of mRNA molecules; and (5) random oligonucleotide probes of varying lengths with high probability of being complementary to relatively large fractions of target molecules.

A number of preferred embodiments of the invention will now be described with reference to the drawings, in which:

Figure 1 shows a linear DNA polymer.
Figures 2A-B illustrate the hydrogen bonding between purine/pyrimidine bases of two anti-parallel DNA strands.
Figure 3 illustrates a short section of a DNA double helix.
Figures 4-7 illustrate the principle of molecular-array-based hybridization assays.
Figure 8A shows a simple four-feature molecular array in which features contain bound oligonucleotides with sequences represented by the letters "P," "Q," "R," and "S."
Figure 8B shows a four-feature molecular array exposed to a sample solution containing various cDNA target molecules.
Figure 8C illustrates target cDNA molecules incorporating red and green chromophores bound to complementary oligonucleotide probes on the surface of a molecular array.
Figure 9 illustrates a calibration set of features included in a molecular array.
Figure 10 illustrates the basis for selecting one type of probe molecule applicable to gene expression experiments conducted on eukaryotic organisms.
Figure 11 illustrates priming of bacterial mRNA for reverse transcription.
Figure 12 shows a plot of log ($signal_{Cy5}$) versus log ($signal_{Cy3}$).

The present invention is directed to methods for calibrating signals generated by analysis of labeled target molecules bound to the surface features of a molecular array so that the concentrations of the target molecules in a sample solution to which the molecular array has been exposed can be inferred from the measured signals. In several embodiments of the present invention, sets of calibrating features are included in each molecular array that produce signals proportional to the concentration of total nucleic acid molecules in a wide range of sample solutions. Different sets of calibration features may be selected and included in molecular arrays by manufacturers of molecular arrays, and an experimentalist may choose for a given experiment a molecular array that includes a suitable calibration set for the sample solutions to which the experimentalist intends to expose the chosen molecular array. Alternatively, molecular arrays may be prepared by experimentalists, with the experimentalists choosing and including suitable calibration features. It is also possible that experimentalists may be able to select and include particular calibration features in manufactured molecular arrays that include positions for calibration features. Identification and employment of broadly applicable sets of calibration features allows for efficient and cost-effective processing of signal data obtained from molecular arrays to produce absolute or relative measured concentrations of target molecules in sample solutions to which the molecular arrays are exposed.

As discussed above with reference to Figures 8A-C, the lack of proportionality between the concentrations of target molecules in sample solutions and signals generated from features directed towards those target molecules within molecular arrays prevents signal data generated by scanning molecular arrays to be used to directly determine concentration levels of target molecules in sample solutions. In the example illustrated in Figures 8A-C, target molecules containing two different types of chromophore labels hybridize to oligonucleotides within the features of a molecular array. Target cDNA copies of the mRNA molecules, labeled with a red chromophore, are hybridized to complementary probes on the surface of the molecular array by exposing the molecular array to a first sample solution, prepared from cells of an organism prior to exposure to a pharmaceutical agent Target cDNA copies of the mRNA molecules, labeled with a green chromophore, are hybridized to the features of the molecular array by exposing the molecular array to a second sample solution prepared from the tissue of the organism following exposure of the organism to a pharmaceutical agent. The relative green-to-red signal ratio for a particular probe is generally related to the relative levels of gene expression for the particular gene generating the target cDNA molecule complementary to the probe molecule. However, as discussed above with reference to Table 3, because of the different relative efficiencies of signal production of the two chromophores, and because of various chemical interactions between target molecules, and other, non-probe molecules, as well as manufacturing defects in the molecular array, the ratio of signal intensities for a particular feature may not, in fact, correspond to the relative levels of gene expression for the gene to which the feature is directed.

One approach to processing signal ratio data is to normalize signals produced by one chromophore to signals produced by another chromophore by dividing each signal produced by a particular label by the geometric mean of all signals produced by the label in scanning of a molecular array. In mathematical terms, the normalization of a particular signal can be expressed as follows:

$$S_{i\ normalized} = \frac{S_i}{\sqrt[N]{\prod_{j=1}^{N} S_j}}$$

*where N = the total number of features from which the particular signal is scanned*

In many cases, the ratio of normalized signals for a particular feature is more closely proportional to the relative concentrations of the target molecules in two samples.

This normalization technique is illustrated, below, continuing with the example illustrated in Figures 8A-C. In Table 4, below, the green-to-red ratios of the actual concentrations of the target molecules corresponding to genes *p* through *s*, provided above in Table 1, are shown:

| | p | q | r | s |
|---|---|---|---|---|
| | 5 | 2 | 0.286 | 1 |

**Table 4**

The green-to-red signal ratios calculated from the hypothetical signal data obtained from the molecular array, provided above in Table 3, is shown below in Table 5:

| | p | q | r | s |
|---|---|---|---|---|
| | 3.45 | 0.77 | 0.19 | 0.67 |

**Table 5**

Comparison of the green-to-red signal ratios in Table 4 and Table 5 again demonstrates the unreliability of unprocessed signal data for determining levels of gene expression. For example, the green-to-red signal ratio calculated based on the observed signals for the *q* gene product seems to indicate that the *q* gene is down-regulated following exposure of the organism to the pharmaceutical agent. However, as shown in Table 1, above, gene *q* was actually up-regulated following exposure of the organism to a pharmaceutical agent.

Table 6, below, provides normalized red and green signals obtained from the observed red and green signals, shown in Table 3, using the normalization formula provided above:

| | p | q | r | s |
|---|---|---|---|---|
| | .17 | 106.84 | 0.24 | 0.24 |
| | 0.75 | 107.45 | 0.06 | 0.21 |

**Table 6**

Table 7, below, provides green-to-red signal ratios calculated from the normalized green and red signals provided in Table 6:

| | p | q | r | s |
|---|---|---|---|---|
| | 4.52 | 1.01 | 0.25 | 0.88 |

**Table 7**

Comparison of the green-to-red signal ratios of Table 7 to those shown in Tables 4 and 5 demonstrates that the normalized signal ratios are more proportional to the actual concentrations of corresponding target molecules in the sample solution. Normalization is particularly effective when the number of up-regulated genes is close to the number of down-regulated genes so that the overall cumulative expression level of genes within the tissue from which sample solutions are prepared is relatively constant. However, in cases where the overall level of gene expression changes in the set of genes sampled, this normalization technique is inadequate for normalizing signal data. Note that such changes can take place either due to an overall increase in gene expression within the organism, or due to sampling bias resulting from measuring a subset of genes with a bias towards up-regulated or down-regulated genes.

Another approach to normalizing different types of signals obtained by instrumental scanning molecular arrays is to employ standard feature sets within each molecular array. Rather than employing mathematical techniques to adjust signals produced from different labels to one another, as in the normalization technique described above, the this technique involves selecting a set of standard features that produce signals with a known correspondence to the nucleic acid content of sample solutions.

Figure 9 illustrates a set of standard features included in a molecular array. In Figure 9, the darkly colored standard features, such as standard feature 902, contain select probe molecules that are complementary to target molecules that are known to be present in a sample solution and that reliably produce signals proportional to the concentrations of their respective target molecules. In any given experiment, a proportionality constant can be determined for the features of the standard set, and then can be applied to signals measured from the remaining features in order to generate sample-solution concentration values from the measured signals of the remaining features. However, this common technique has serious deficiencies. First, a standard feature set valid for one type of sample solution may be completely inadequate for another type of sample solution. For example, the target molecules of standard features within a first type of sample solution may not associate with any other molecules within the sample solution and may therefore have effective concentrations for hybridization with probe molecules equal to their absolute concentrations. However, in a second sample solution, a significant number of the target molecules of the standard set may associate with other molecules in the second sample solution not present in the first sample solution to lower the target molecules' effective concentration for hybridization with standard set probe molecules. Thus, the proportionality constant determined from signals produced by the standard feature set upon exposure of a molecular array to the second sample solution may greatly exceed the actual proportionality constant based on the true concentration of the target molecules in the second sample solution. Standard feature sets valid over only small ranges of different types of sample solutions are costly, requiring time-consuming and expensive research efforts to identify suitable standard probes that can be amortized over only a small percentage of possible assays. A second deficiency of commonly-employed standard set methods, in the case of gene expression experiments, is that the standard feature set should be directed towards the transcription products of housekeeping genes or, in other words, genes that are generally not up-regulated or down-regulated during the time frames over which samples are prepared. However, it is becoming increasingly evident that the expression levels of many genes formerly considered to be housekeeping genes do, in fact, fluctuate over time or in response to changing experimental conditions. If the transcripts of target molecules for standard feature set probes fluctuate with respect to non-calibration-feature-set probes, then the proportionality constant calculated based on the standard feature set signals may incorrectly amplify or depress calculated concentrations or non-calibration-feature-set signals to which the proportionality constant is applied.

To overcome the deficiencies of the mathematical normalization techniques and the deficiencies of common standard-feature-set techniques, embodiments of the present invention rely on determining and employing calibration feature sets containing probe molecules that reliably hybridize to large fractions of all target molecules in a wide range of sample solution types. If the overall response of the system is linear, then a probe molecule calibration set can be used to normalize signals as follows. The average signal measured for a calibration feature subset can be approximated as the product of a response constant particular to a given label and instrumental analysis technique, the mole fraction of labeled sample molecules that hybridize to the features of the calibration feature subset, and to the amount of nucleic acid in the sample solution to which a molecular array has been exposed prior to analysis, by the following expression:

$$S_N \cong R\, X_N M_{NA}$$

*where* $N$ *= number of features in calibration subset,* $\{S_{j1}, S_{j2}, S_{j3} \ldots S_{jN}\}$

$S_M$ *= average signal of subset features,*

$$\frac{1}{N}\sum_{i=j_1}^{j_N} S_i$$

*R = response constant*
$X_N$ *= mole fraction of labeled sample molecules that hybridize to features of the calibration feature subset*
$M_{NA}$ *= amount of nucleic acid in the sample*

Similarly, the signal measured for a particular feature can be expressed in terms of a response constant, mole fraction, and the amount of nucleic acid in the sample solution as follows:

$$S_i = RX_iM_{NA}$$

*where*

$S_i$ *= signal measured for feature i*
$X_i$ *= mole fraction of labeled sample molecules that hybridize to feature i*
$M_{NA}$ *= amount of nucleic acid in the sample*

A normalized signal intensity, $\lambda_i$, can be defined as follows:

$$\lambda_i = \frac{S_i}{S_N}$$

By replacing $S_i$ and $S_N$ in the above formula with equivalent expressions from previous formulas, and canceling common terms form the numerator and divisor, the normalized signal intensity $\lambda_i$ can be expressed by the ratio of the mole fraction of the labeled target molecule to which feature i is directed divided by the mole fraction of the labeled sample molecules that hybridize to features of the calibration feature subset as follows:

$$\lambda_i = \frac{S_i}{S_N} = \frac{RX_iM_{NA}}{RX_NM_{NA}} = \frac{X_i}{X_N}$$

An important attribute of a properly chosen calibration feature subset according to the present invention is that the average signal generated by the calibration feature subset is proportional to the total nucleic acid content of any given sample solution for which the calibrated feature subset is valid. When suitable calibration feature subsets having this property are identified, the calibration feature subsets can be employed over a broad range of sample solutions.

Four different types of suitable probe molecules for calibration feature subsets underlie four different embodiments of the present invention. Figure 10 illustrates a basis for selecting one type of probe molecule applicable to gene expression experiments conducted on eukaryotic organisms. Most mRNA transcripts in eukaryotic organisms have the form of mRNA transcript 1002 in Figure 10. The 5' end of the transcript 1004 is a cap region consisting of a methylated guanylate nucleotide linked to the next nucleotide in the mRNA via a 5'-5' triphosphate linkage. The second and third nucleotides from the cap region may also be methylated. A translatable gene sequence 1006 follows the cap region, which is followed by a poly(A) tail 1008, added following transcription, comprising several hundred adenosine nucleotide residues. Reverse transcription of such eukaryotic mRNAs is primed with a poly(T) primer 1010 complementary to the poly(A) tail 1008. Reverse transcriptase synthesizes a cDNA complement 1012 to the coding sequence 1006 of the mRNA starting from the 3' end of the poly(T) primer. The cDNA product of reverse transcription of the mRNA may be amplified through the polymerase chain reaction and labeled to produce the labeled target molecules to which probe molecules within features of a molecular array are directed. Oligonucleotide probe molecules consisting of various lengths of adenosine nucleotides complementary to the 5' poly(T) tails of cDNA copies of the mRNA will thus hybridize, with hybridization potentials, or $T_M$'s, proportional to the length of the poly(A) oligonucleotides within a range of poly(A) oligonucleotide lengths, to almost all cDNA transcripts in any given sample solution. Thus, poly(A) oligonucleotide probes reliably produce signals proportional to the total concentration of labeled cDNA molecules in sample solutions prepared from eukaryotic mRNAs.

Often, in gene expression experiments, signal strengths vary over several orders of magnitude. Response functions relating measured signal strength to the density of labeled target molecules of the surface of features may be non-linear over the range of measured signal strengths. Because poly(A) oligonucleotide probes of different lengths bind to cDNA transcripts with different affinities, use of a variety of different lengths of poly(A) oligonucleotide probes in a calibration set produces a calibration feature set producing wide range of signal intensities, allowing calculation of average calibration signals for a number of different ranges of signal intensities and thereby providing reasonable calibration of measured signals for signal intensity ranges over which instrument response curves are non-linear.

An important variant of the poly(A) probe can be utilized in the case where the primer used to initiate reverse-transcription of the original mRNA is of the form

$$5'\text{-}[F_1]\text{-}[F_2]\text{-}(T)_n\text{-}VN\text{-}3'$$

where $F_1$ is a sequence that does not end up in the final, labeled product (e.g. a T7 RNA polymerase promoter, used for in vitro linear amplification of the original cDNA into cRNA), $F_2$ is a sequence that does end up in the final, labeled product (e.g. a promoter extension placed after the start-of-transcription base, used to increase the efficiency of transcription elongation), $(T)_n$ is a poly(T) stretch, and the sequence "VN" indicates that the penultimate base is an equimolar combination of the bases A, G and C, while the 3' base can be A, G, C or T. The sequence "VN" assures that reverse transcription is initiated at the junction of the poly(A) tail and the mRNA-transcribed 3' end. In this case, probes of the general form

$$5'\text{-}(A)_m\text{-}[F_2]_c\text{-}X\text{-surface}$$

may be used, where $m \leq n$, $[F_2]_c$ is the Watson-Crick complementary sequence of $[F_2]$, and X is an optional linker sequence that spaces the rest of the probe away from the array surface.

A second type of probe conforming to the criteria of the present invention, useful for gene expression experiments based on human tissue samples, is prepared by synthesizing probe molecules complementary to the cDNA transcripts of the common Alu sequences found in many different human genes. The common Alu sequence is related to the sequence of 7SRNA, a component of an RNA signal recognition particle. Because Alu sequences frequently occur in the human genome, probe oligonucleotides complementary to cDNA transcripts of Alu sequences can be expected to hybridize with a large fraction of labeled target molecules in any sample solution containing cDNA transcripts of mRNA extracted from human tissues.

Bacterial mRNAs generally do not contain 3' poly(A) tails. In order to prepare cDNA transcripts of bacterial mRNA, short oligonucleotide primers complementary to the 5' terminal sequences of bacterial mRNAs are introduced into a bacterial mRNA solution to produce short regions of terminal hybrid duplex to the primer strand of which reverse transcriptase begins appending nucleotides complementary to the nucleotide residues of bacterial mRNA. Figure 11 illustrates priming of bacterial mRNA. The bacterial mRNA 1102 hybridizes with a short primer 1004 complementary to the 3' terminal sequence 1106 of the bacterial mRNA. A probe oligonucleotide that can hybridize to a large fraction of the total cDNA transcripts generated from bacterial mRNAs can be created as a complement to a short 5' synthetic sequence 1108 appended to the 5' end of the bacterial primer 1104. If the common synthetic sequence 1108 is added to all bacterial primers, then the complementary probe will hybridize to all target cDNA molecules produced from the bacterial mRNAs. Thus, a probe molecule complementary to this synthetic sequence can hybridize to any cDNA produced by reverse transcription of bacterial mRNAs in any sample solution. As with the poly(A) oligonucleotide probes described with reference to Figure 10, the length of the synthetic sequence in corresponding probe oligonucleotides may be varied to produce probes that generate different signal intensities to allow for normalization over ranges of signal intensities spanning non-linear instrument response curves. Furthermore, this technique may also be employed in non-bacterial mRNA systems.

Finally, probe molecules suitable for calibration feature sets conforming to the criteria required by the present invention can be random oligonucleotide sequences. The random oligonucleotide sequences can be synthesized by including all four deoxynucleotide triphosphates at each elongation step in oligonucleotide probe synthesis. Each feature of the calibration feature set will thus contain a large number of copies of all possible random sequences of a given length. Such features can be expected to hybridize to a large fraction of possible labeled target molecules in any given sample solution.

The calibration feature set features may be dispersed systematically over the area of a molecular array, as illustrated in Figure 9, to measure systematic gradients in the signal across the array. This measurement can be used to detect and correct the effects of manufacturing defects in which densities of probe molecules within features vary systematically over the surface of the array. In addition, this measurement can be used to detect and correct for gradients of signal caused by scanner focus problems. Generally, by computing and using signal ratios, problems caused by signal gradients can be avoided or implicitly taken into account. However, when the gradients, or, in other words, slopes of systematic increase or decrease in signal strength vary for different types of signals, the computed ratios are no longer insensitive to the systematic variations of signal strength across an array. In such cases, the calibration sets of the present invention can be used to calibrate measured signal intensities to initial solution concentrations of target molecules. Calibration feature sets of many different sizes may be employed relative to the size of the molecular arrays in which they are included. Relatively larger calibration feature sets may provide more reliable average signal intensities at the expense of less surface area devoted to non-calibration-feature-set features. Particular probe molecules can be redundantly incorporated into a number of calibration-feature-set features in order to further increase the reliability of the calibrated feature set and to internally measure variability of signal intensity within the calibration feature set. The average intensity measured over a calibrated feature set may provide, on a per label type basis, an independent determination of the total nucleic acid content of a sample solution applied to a molecular array.

Experimental verification of the first of the four above-described embodiments employing poly(A) oligonucleotide probes was obtained as follows, Purified mRNA from human K-562 cells was amplified and labeled to produce labeled cRNA target molecules by a method disclosed in U.S. Patent Application No. 09/322692, entitled "A Method for Linear Amplification of Heterogeneous mRNA" and filed May 28, 1999. A sample solution containing equal concentrations of Cy3- and Cy5-labeled K-562 cRNA was prepared and applied to two molecular arrays, each containing probes to about 100 human reference mRNA sequences. Approximately nine probes for each reference sequence were included in the two molecular arrays, each probe redundantly included in a sufficient number of different features to fill the molecular arrays. In addition, the two molecular arrays also contained four features per array containing each of the poly(A) normalization probes shown in Table 8, below:

| Probe | Length | Sequence (5' -> 3') | Total Replicates | SEQ ID |
|---|---|---|---|---|
| T7T18Apad_PS27-20-0003 | 20 | AAAAAAAAAAAAAAAATCTC | 8 | 1 |
| T7T18Apad_PS26-21-0003 | 21 | AAAAAAAAAAAAAAAATCTCC | 8 | 2 |
| T7T18Apad_PS25-22-0003 | 22 | AAAAAAAAAAAAAAAATCTCCC | 8 | 3 |
| T7T18Apad_PS24-23-0003 | 23 | AAAAAAAAAAAAAAAATCTCCCA | 8 | 4 |
| T7T18Apad_PS13-23-0001 | 23 | AAAAAAAAAAAAAAAAAATCTCC | 8 | 5 |
| T7T18Apad_PS23-24-0003 | 24 | AAAAAAAAAAAAAAAATCTCCCAA | 8 | 6 |
| T7T18Apad_PS12-24-0001 | 24 | AAAAAAAAAAAAAAAAAATCTCCC | 8 | 7 |
| T7T18Apad_PS22-25-0003 | 25 | AAAAAAAAAAAAAAAATCTCCCAAA | 8 | 8 |
| T7T18Apad_PS11-25-0001 | 25 | AAAAAAAAAAAAAAAAAATCTCCCA | 8 | 9 |
| T7T18Apad_PS21-26-0003 | 26 | AAAAAAAAAAAAAAAATCTCCCAAAA | 8 | 10 |
| T7T18Apad_PS10-26-0001 | 26 | AAAAAAAAAAAAAAAAAATCTCCCAA | 8 | 11 |
| T7T18Apad_PS9-27-0001 | 27 | AAAAAAAAAAAAAAAAAATCTCCCAAA | 8 | 12 |
| T7T18Apad_PS20-27-0003 | 27 | AAAAAAAAAAAAAAAATCTCCCAAAAA | 8 | 13 |
| T7T18Apad_PS8-28-0001 | 28 | AAAAAAAAAAAAAAAAAATCTCCCAAAA | 8 | 14 |
| T7T18Apad_PS7-28-0001 | 28 | AAAAAAAAAAAAAAAAAATCTCCCAAAA | 8 | 15 |
| T7T18Apad_PS19-28-0003 | 28 | AAAAAAAAAAAAAAAATCTCCCAAAAAA | 8 | 16 |
| T7T18Apad_PS6-29-0001 | 29 | AAAAAAAAAAAAAAAAAATCTCCCAAAAA | 8 | 17 |
| T7T18Apad_PS18-29-0003 | 29 | AAAAAAAAAAAAAAAATCTCCCAAAAAAA | 8 | 18 |
| T7T18Apad_PS5-30-0001 | 30 | AAAAAAAAAAAAAAAAAATCTCCCAAAAAA | 8 | 19 |
| T7T18Apad_PS17-30-0003 | 30 | AAAAAAAAAAAAAAAATCTCCCAAAAAAAA | 8 | 20 |
| T7T18Apad_PS4-31-0001 | 31 | AAAAAAAAAAAAAAAAAATCTCCCAAAAAAA | 8 | 21 |
| T7T18Apad_PS16-31-0003 | 31 | AAAAAAAAAAAAAAAATCTCCCAAAAAAAAA | 8 | 22 |
| T7T18Apad_PS3-32-0001 | 32 | AAAAAAAAAAAAAAAAAATCTCCCAAAAAAAA | 8 | 23 |
| T7T18Apad_PS15-32-0003 | 32 | AAAAAAAAAAAAAAAATCTCCCAAAAAAAAAA | 8 | 24 |
| T7T18Apad_PS2-33-0001 | 33 | AAAAAAAAAAAAAAAAAATCTCCCAAAAAAAAA | 8 | 25 |
| T7T18Apad_PS14-33-0003 | 33 | AAAAAAAAAAAAAAAATCTCCCAAAAAAAAAAA | 8 | 26 |
| T7T18Apad_PS1-34-0001 | 34 | AAAAAAAAAAAAAAAAAATCTCCCAAAAAAAAAA | 8 | 27 |
| T7T18Apad_PS0-35-0001 | 35 | AAAAAAAAAAAAAAAAAATCTCCCAAAAAAAAAAA | 8 | 28 |

**Table 8**

The target Cy3- and Cy5-labeled K-562 cRNA molecules were allowed to hybridize to probe molecules on the surface of the two molecular arrays, the sample solution was removed, and the two molecular arrays were scanned to produce measured Cy3 and Cy5 signal intensities. The measured signal intensities from redundant features or, in other words, features all containing the same probe molecule, were averaged. The logs of the average Cy3 and Cy5 signals measured for the normalization probes are shown below, in Table 9:

Table 9

| Probe | Log (average Cy3 Signal) | Log (average Cy5 Signal) |
|---|---|---|
| T7T18Apad_PS27-20-0003 | 3.206 | 3.522 |
| T7T18Apad_PS26-21-0003 | 3.505 | 3.831 |
| T7T18Apad_PS25-22-0003 | 3.705 | 4.045 |
| 17T18Apad_PS24-23-0003 | 3.826 | 4.177 |
| T7T18Apad_PS13-23-0001 | 3.795 | 4.141 |
| T7T18Apad_PS23-24-0003 | 3.911 | 4.270 |
| T7T18Apad_PS12-24-0001 | 3.880 | 4.240 |
| T7T18Apad_PS22-25-0003 | 3.949 | 4.320 |
| T7T18Apad_PS11-25-0001 | 3.936 | 4.296 |
| T7T18Apad_PS21-26-0003 | 3.975 | 4.353 |
| T7T18Apad_PS10-26-0001 | 3.954 | 4.323 |
| T7T18Apad_PS9-27-0001 | 3.965 | 4.330 |
| T7T18Apad_PS20-27-0003 | 3.990 | 4.374 |
| T7T18Apad_PS8-28-0001 | 3.988 | 4.354 |
| T7T18Apad_PS7-28-0001 | 3.981 | 4.350 |
| T7T18Apad_PS19-28-0003 | 3.997 | 4.384 |
| T7T18Apad_PS6-29-0001 | 4.012 | 4.380 |
| T7T18Apad_PS18-29-0003 | 4.015 | 4.404 |
| T7T18Apad_PS5-30-0001 | 4.031 | 4.422 |
| T7T18Apad_PS17-30-0003 | 4.030 | 4.415 |
| T7T18Apad_PS4-31-0001 | 4.021 | 4.401 |
| T7T18Apad_PS16-31-0003 | 4.041 | 4.424 |
| T7T18Apad_PS3-32-0001 | 4.027 | 4.408 |
| T7T18Apad_PS15-32-0003 | 4.034 | 4.420 |
| T7T18Apad_PS2-33-0001 | 4.021 | 4.407 |
| T7T18Apad_PS14-33-0003 | 4.033 | 4.421 |
| T7T18Apad_PS1-34-0001 | 4.019 | 4.401 |
| T7T18Apad_PS0-35-0001 | 4.030 | 4.409 |

The relationship between log Cy3 signal intensities and log Cy5 signal intensities was determined via linear regression as:

$$\log(\mathrm{signal}_{Cy5}) = 1.095 \log(\mathrm{signal}_{Cy3}) - .003$$

where $\mathrm{signal}_{Cy5}$ and $\mathrm{signal}_{Cy3}$ indicate the average signal intensity for any given probe. This linear relationship between logs of average Cy5 and Cy3 intensities indicates the following relationship between measured Cy5 intensities and measured Cy3 intensities:

$$signal_{Cy5}=.99\ signal^{1.095}$$

The same K-562 mRNA solution was used to prepare the Cy3 and Cy5-labeled cRNA target molecule solutions that were applied to the molecular arrays. Thus, straightforward normalization of the measured Cy3 and Cy5 signal intensities should produce normalized Cy5 and normalized Cy5 signal intensities that are equal for each probe or molecular array feature. Thus, a correct normalization function can be back calculated from the measured signal intensities. This normalization function was calculated from the measured signal intensities of the one hundred reference human mRNA sequences as follows:

$$\log(signal_{Cy5}) = 1.064 \log(signal_{Cy3}) + .146$$

Figure 12 shows a plot of log ($signal_{Cy5}$) versus log ($signal_{Cy3}$). Note that the linear relationship between log ($signal_{Cy5}$) and log ($signal_{Cy3}$) for the general gene-specific probes coincides quite well with the ratios for the normalization probes, graphically illustrating the closeness of the two derived equations relating log ($signal_{Cy5}$) and log ($signal_{Cy3}$) for the gene-specific probe data and the normalization probe data, above.

Although the present invention has been described in terms of a particular embodiment, it is not intended that the invention be limited to this embodiment. Modifications within the spirit of the invention will be apparent to those skilled in the art. In particular, calibration feature sets can be constructed according to the criteria of the present invention for sample solutions containing many different types of labeled target molecules. As described above, a suitable probe for a calibrated-feature-set feature is a probe molecule that binds to a large fraction of labeled target molecules over a wide range of sample solutions to which a molecular array may be exposed. Thus, in an antigen detecting molecular array system, where antibody probes are bound to the features of the molecular array, a very indiscriminate and promiscuously binding antibody that binds to a whole class of antigens may be selected as a suitable probe for a calibration-feature-set feature. As pointed out above, many different sizes of calibration feature sets relative to the sizes of the molecular arrays in which they are included may be employed, and the features of the calibration feature set maybe positioned over the surface of the molecular array in different ways in order to account for potential manufacturing defects and experimental conditions. A calibration feature may contain a single type of molecule, or may contain a number of different types of molecules. As discussed above, calibration feature sets may be included by manufacturers or included by experimentalists in manufactured molecular arrays or in molecular arrays fabricated by the experimentalists.

The foregoing description, for purposes of explanation, used specific nomenclature to provide a thorough understanding of the invention. However, it will be apparent to one skilled in the art that the specific details are not required in order to practice the invention. In other instances, well-known circuits and devices are shown in block diagram form in order to avoid unnecessary distraction from the underlying invention. Thus, the foregoing descriptions of specific embodiments of the present invention are presented for purposes of illustration and description; they are not intended to be exhaustive or to limit the invention to the precise forms disclosed, obviously many modifications and variations are possible in view of the above teachings. The embodiments were chosen and described in order to best explain the principles of the invention and its practical applications and to thereby enable others skilled in the art to best utilize the invention and various embodiments with various modifications as are suited to the particular use contemplated. It is intended that the scope of the invention be defined by the following claims and their equivalents:

SEQUENCE LISTING

<110> AGILENT TECHNOLOGIES

<120> CALIBRATION OF MOLECULAR ARRAYS CONTAINING NUCLEIC ACID PROBES

<130> 1013

<140>
<141>

<160> 28

<170> PatentIn Ver. 2.0

<210> 1
<211> 20
<212> DNA
<213> Artificial Sequence

<220>
<223> Description of Artificial Sequence:probes to target sequences

<400> 1
aaaaaaaaaa aaaaaatctc 20

<210> 2
<211> 21
<212> DNA
<213> Artificial Sequence

<220>
<223> Description of Artificial Sequence:probes to target sequences

<400> 2
aaaaaaaaaa aaaaaatctc c 21

<210> 3
<211> 22
<212> DNA
<213> Artificial Sequence

<220>
<223> Description of Artificial Sequence:probes to target sequences

<400> 3
aaaaaaaaaa aaaaaatctc cc 22

<210> 4
<211> 23
<212> DNA
<213> Artificial Sequence

<220>
<223> Description of Artificial Sequence:probes to target sequences

<400> 4
aaaaaaaaaa aaaaaatctc cca 23

<210> 5
<211> 23
<212> DNA
<213> Artificial Sequence

<220>
<223> Description of Artificial Sequence:probes to target sequences

<400> 5
aaaaaaaaaa aaaaaaaatc tcc 23

<210> 6
<211> 24
<212> DNA
<213> Artificial Sequence

<220>
<223> Description of Artificial Sequence:probes to target sequences

<400> 6
aaaaaaaaaa aaaaaatctc ccaa 24

<210> 7
<211> 24
<212> DNA
<213> Artificial Sequence

<220>
<223> Description of Artificial Sequence:probes to target sequences

<400> 7
aaaaaaaaaa aaaaaaaatc tccc 24

<210> 8
<211> 25
<212> DNA
<213> Artificial Sequence

<220>
<223> Description of Artificial Sequence:probes to target sequences

<400> 8
aaaaaaaaaa aaaaaatctc ccaaa 25

<210> 9
<211> 25

<212> DNA
<213> Artificial Sequence

<220>
<223> Description of Artificial Sequence:probes to target sequences

<400> 9
aaaaaaaaaa aaaaaaaatc tccca 25

<210> 10
<211> 26
<212> DNA
<213> Artificial Sequence

<220>
<223> Description of Artificial Sequence:probes to target sequences

<400> 10
aaaaaaaaaa aaaaaatctc ccaaaa 26

<210> 11
<211> 26
<212> DNA
<213> Artificial Sequence

<220>
<223> Description of Artificial Sequence:probes to target sequences

<400> 11
aaaaaaaaaa aaaaaaaatc tcccaa 26

<210> 12
<211> 27
<212> DNA
<213> Artificial Sequence

<220>
<223> Description of Artificial Sequence:probes to target sequences

<400> 12
aaaaaaaaaa aaaaaaaatc tcccaaa 27

<210> 13
<211> 27
<212> DNA
<213> Artificial Sequence

<220>
<223> Description of Artificial Sequence:probes to target sequences

<400> 13
aaaaaaaaaa aaaaaatctc ccaaaaa 27

<210> 14
<211> 28
<212> DNA
<213> Artificial Sequence

<220>
<223> Description of Artificial Sequence:probes to target sequences

<400> 14
aaaaaaaaaa aaaaaaaatc tcccaaaa 28

<210> 15
<211> 28
<212> DNA
<213> Artificial Sequence

<220>
<223> Description of Artificial Sequence:probes to target sequences

<400> 15
aaaaaaaaaa aaaaaaaatc tcccaaaa 28

<210> 16
<211> 28
<212> DNA
<213> Artificial Sequence

<220>
<223> Description of Artificial Sequence:probes to target sequences

<400> 16
aaaaaaaaaa aaaaaatctc ccaaaaaa 28

<210> 17
<211> 29
<212> DNA
<213> Artificial Sequence

<220>
<223> Description of Artificial Sequence:probes to target sequences

<400> 17
aaaaaaaaaa aaaaaaaatc tcccaaaaa 29

<210> 18
<211> 29
<212> DNA
<213> Artificial Sequence

<220>
<223> Description of Artificial Sequence:probes to target sequences

<220>
<223> Description of Artificial Sequence:probes to target sequences

<400> 23
aaaaaaaaaa aaaaaaaatc tcccaaaaaa aa 32

<210> 24
<211> 32
<212> DNA
<213> Artificial Sequence

<220>
<223> Description of Artificial Sequence:probes to target sequences

<400> 24
aaaaaaaaaa aaaaaatctc ccaaaaaaaa aa 32

<210> 25
<211> 33
<212> DNA
<213> Artificial Sequence

<220>
<223> Description of Artificial Sequence:probes to target sequences

<400> 25
aaaaaaaaaa aaaaaaaatc tcccaaaaaa aaa 33

<210> 26
<211> 33
<212> DNA
<213> Artificial Sequence

<220>
<223> Description of Artificial Sequence:probes to target sequences

<400> 26
aaaaaaaaaa aaaaaatctc ccaaaaaaaa aaa 33

<210> 27
<211> 34
<212> DNA
<213> Artificial Sequence

<220>
<223> Description of Artificial Sequence:probes to target sequences

<400> 27
aaaaaaaaaa aaaaaaaatc tcccaaaaaa aaaa 34

<210> 28
<211> 35

<212> DNA
<213> Artificial Sequence

<220>
<223> Description of Artificial Sequence:probes to target sequences

<400> 28
aaaaaaaaaa aaaaaaaatc tcccaaaaaa aaaaa 35

## Claims

1. A method for calibrating data scanned from a molecular array, the method comprising:

    selecting a molecular array (900) that includes a set of calibrating probes that hybridize to a sufficient fraction of target molecules in sample solutions to which the molecular array (900) is intended to be exposed to produce corresponding signal intensities upon reading of the calibrating probes proportional to the total concentration of target molecules in the sample solutions;
    exposing the molecular array (900) to a sample solution;
    reading the molecular array (900) to determine signal intensities for each feature of the molecular array;
    calculating a collective calibration signal intensity from the signal intensities read from the set of calibrating features (902-906); and
    calculating normalized signal intensities based on signal intensities read from features of the molecular array by applying to the signal intensities a normalization function that includes the calculated collective calibration signal.

2. A method as claimed in claim 1 wherein probes contained in the molecular array (900) and calibrating probes are selected from among:

    oligonucleotide probes complementary to cDNA copies of cDNA transcripts of eukaryotic mRNA molecules and poly(A) oligonucleotide calibrating probes;
    oligonucleotides probes complementary to cRNA copies complementary to eukaryotic mRNA molecules and poly(A) oligonucleotide calibrating probes;
    oligonucleotide probes complementary to cDNA copies of cDNA transcripts of human mRNA molecules and oligonucleotide calibrating probes complementary to cDNA transcripts of Alu repeat sequences common to many human mRNAs;
    oligonucleotide probes complementary to cDNA copies of the mRNA molecules and oligonucleotide calibrating probes complementary to a synthetic nucleotide sequence appended to primers for reverse transcription of the mRNA molecules; and
    oligonucleotide probes complementary to cDNA copies of the mRNA molecules and random-sequence oligonucleotide probes.

3. A method as claimed in claim 1 or 2,
    wherein calculating a collective calibration signal intensity from the signal intensities read from the set of calibrating features (902-906) further includes calculating a set of collective calibration signal intensities by partitioning the signal intensities generated from the set of calibrating features into sets of similar calibrating signal intensities and calculating a collective signal intensity for each set, so that the sets of similar calibrating signal intensities each covers a discrete range of signal intensities and so that the discrete ranges of signal intensities span an overall range of signal intensities generated from features of the molecular array (900), and
    wherein calculating normalized signal intensities based on signal intensities read from features of the molecular array by applying to the signal intensities a normalization function that includes the calculated collective calibration signal further includes applying to each signal intensity a normalization function that includes the calculated collective calibration signal calculated from the set of calibrating signal intensities within the discrete range of intensities in which the signal intensity generated from the feature of the molecular array is included.

**4.** A method as claimed in claim 1 or claim 2

wherein calculating a collective calibration signal intensity from the signal intensities read from the set of calibrating features (902-906) further includes calculating the average calibration signal intensity from the signal intensities read from the set of calibrating features, and

wherein calculating normalized signal intensities based on signal intensities read from features of the molecular array (900) by applying to the signal intensities a normalization function that includes the calculated collective calibration signal further includes dividing each signal intensity by the calculated average calibration signal intensity.

**5.** A method as claimed in claim 1 or claim 2

wherein calculating a collective calibration signal intensity from the signal intensities read from the set of calibrating features (902-906) further includes calculating the mean calibration signal intensity from the signal intensities read from the set of calibrating features, and

wherein calculating normalized signal intensities based on signal intensities read from features of the molecular array (900) by applying to the signal intensities a normalization function that includes the calculated collective calibration signal further includes dividing each signal intensity by the calculated mean calibration signal intensity.

**6.** A molecular array (900) containing features that each include probe molecules, the molecular array incorporating a calibration feature set (902-906), the calibration feature set comprising features containing calibration probe molecules, the calibration probe molecules incorporated into each calibration feature capable of hybridizing to a sufficient fraction of target molecules in a number of different types of sample solutions to produce corresponding signal intensities upon scanning of the calibrating probe molecules proportional to the total concentration of target molecules in the different types of sample solutions.

**7.** A molecular array (900) as claimed in claim 6 in which each calibration feature (902-906) contains a single type of calibration probe molecule.

**8.** A molecular array (900) as claimed in claim 6 in which each calibration feature (902-906) contains a number of different types of calibration probe molecule.

**9.** A molecular array (900) as claimed in claim 6 wherein a number of calibration features each contains a single type of calibration probe molecule and wherein a number of calibration features each contains a number of different types of calibration probe molecules.

**10.** A molecular array (900) as claimed in any of claims 6 to 9 wherein probe molecules and calibration probes are selected from among:

oligonucleotide probes complementary to cDNA copies of cDNA transcripts of eukaryotic mRNA molecules and poly(A) oligonucleotide calibrating probes;
oligonucleotide probes complementary to cRNA copies complementary to eukaryptic mRNA molecules and poly(A) oligonucleotide calibrating probes;
oligonucleotide probes complementary to cDNA copies of cDNA transcripts of human mRNA molecules and oligonucleotide calibrating probes complementary to cDNA transcripts of Alu repeat sequences common to many human mRNAs;
oligonucleotide probes complementary to cDNA copies of the mRNA molecules and oligonucleotide calibrating probes complementary to a synthetic nucleotide sequence appended to primers for reverse transcription of the mRNA molecules; and
oligonucleotide probes complementary to cDNA copies of the mRNA molecules and random-sequence oligonucleotide calibrating probes.

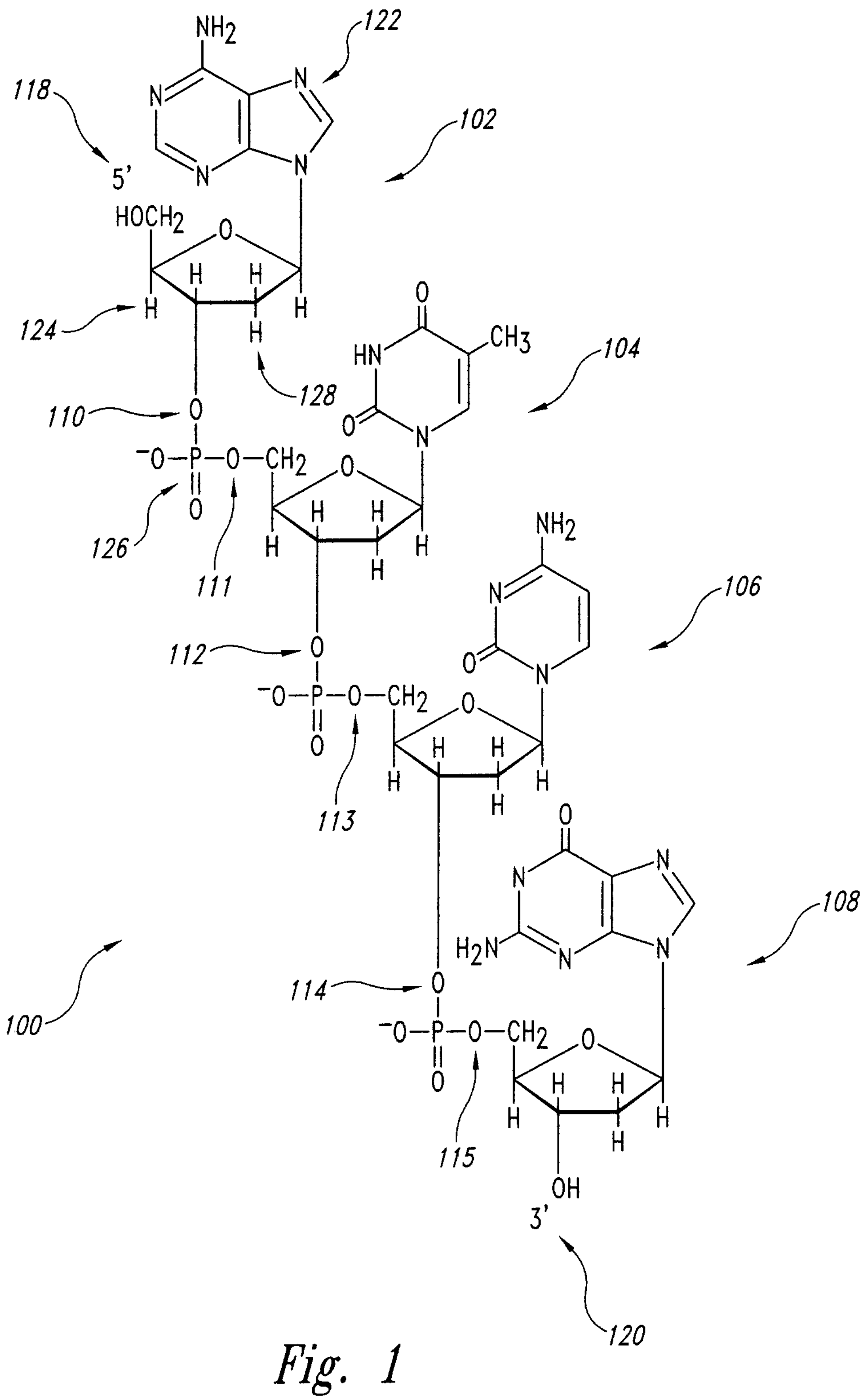

*Fig. 1*

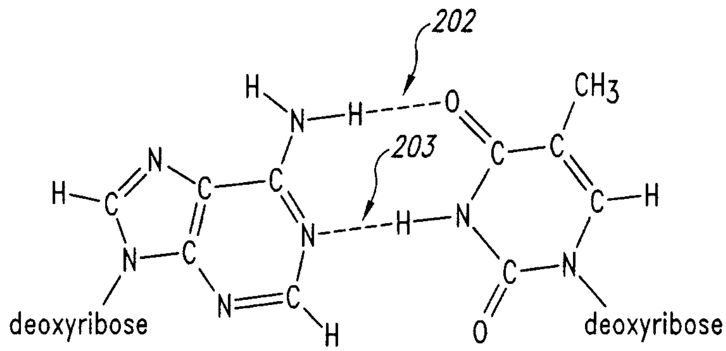


*Fig. 2A*

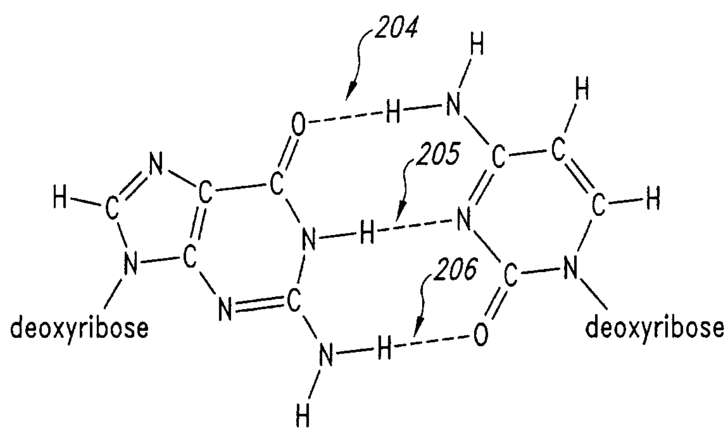


*Fig. 2B*

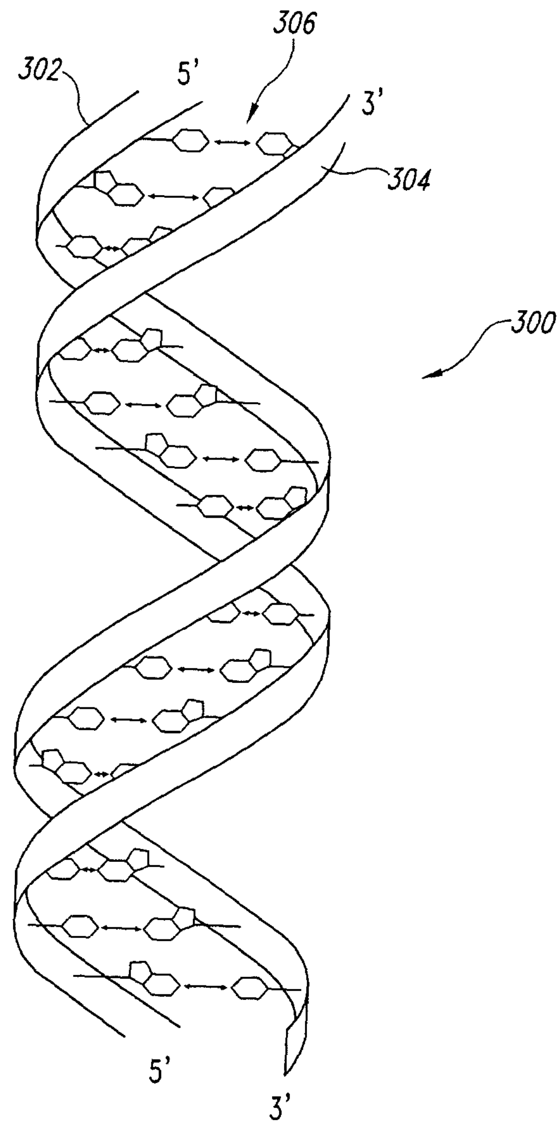


Fig. 3

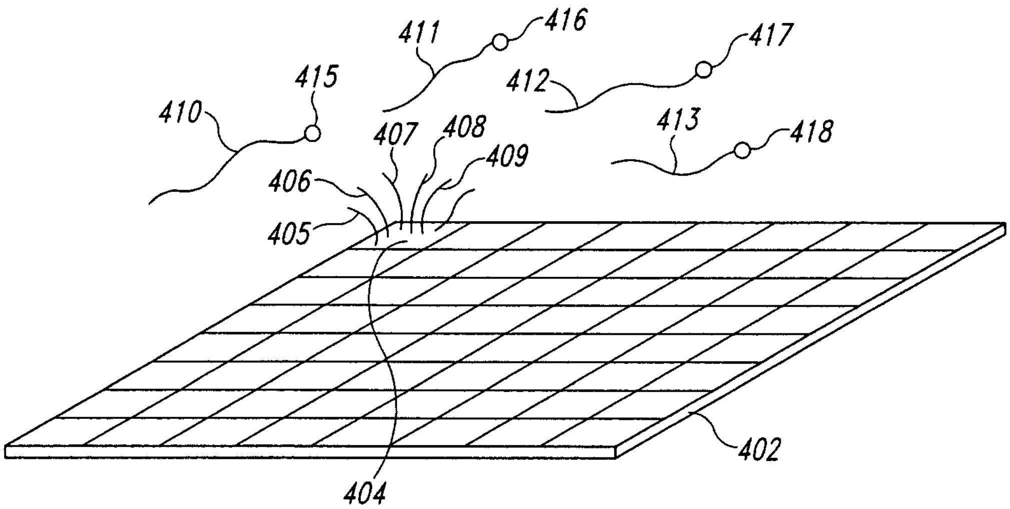
411
416
415
417
410
412
407
408
413
418
409
406
405
402
404

*Fig. 4*

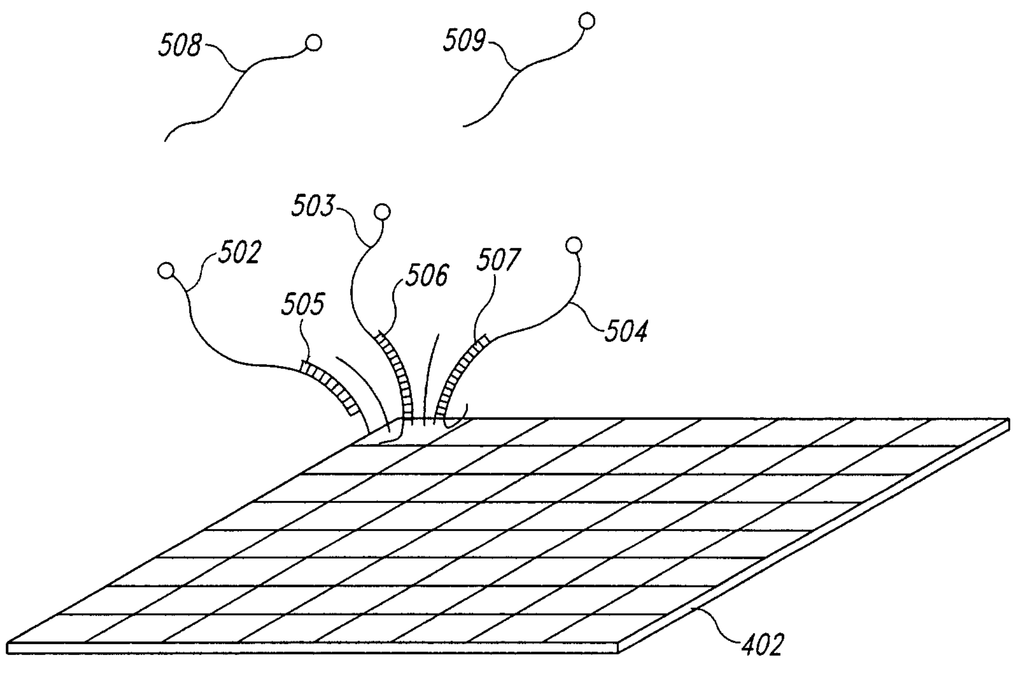
508
509
503
502
506
507
505
504
402

*Fig. 5*

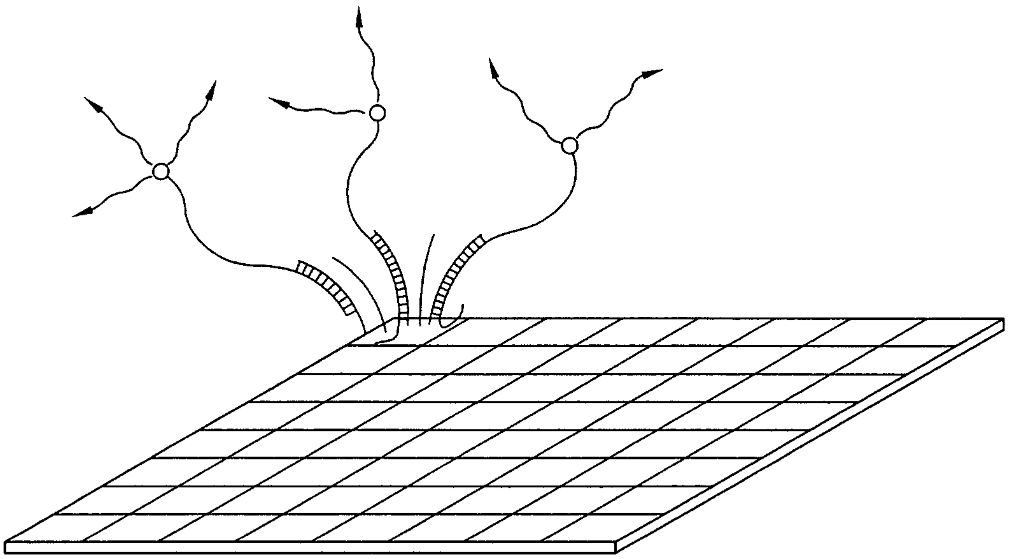

Fig. 6

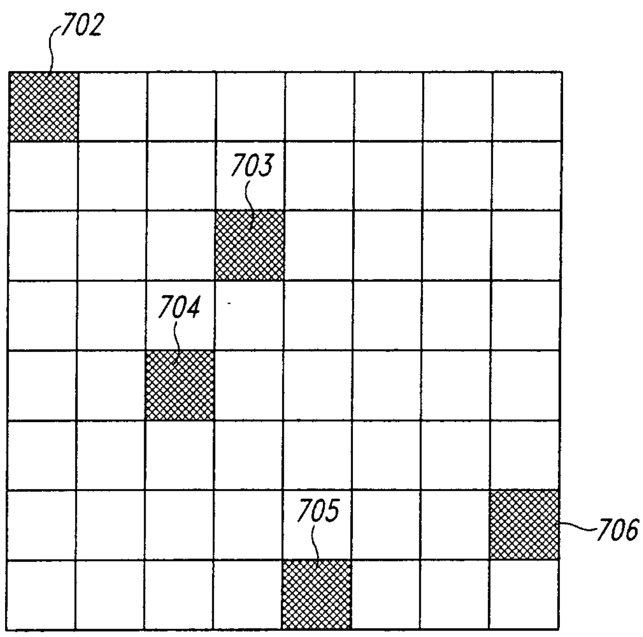


Fig. 7

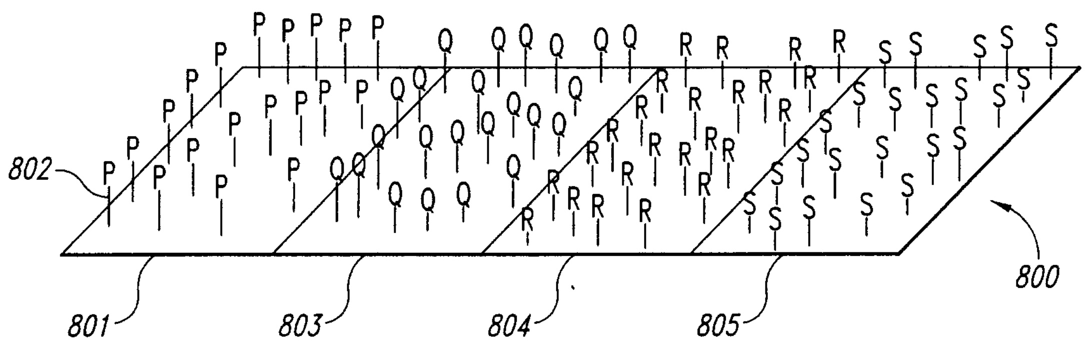


Fig. 8A

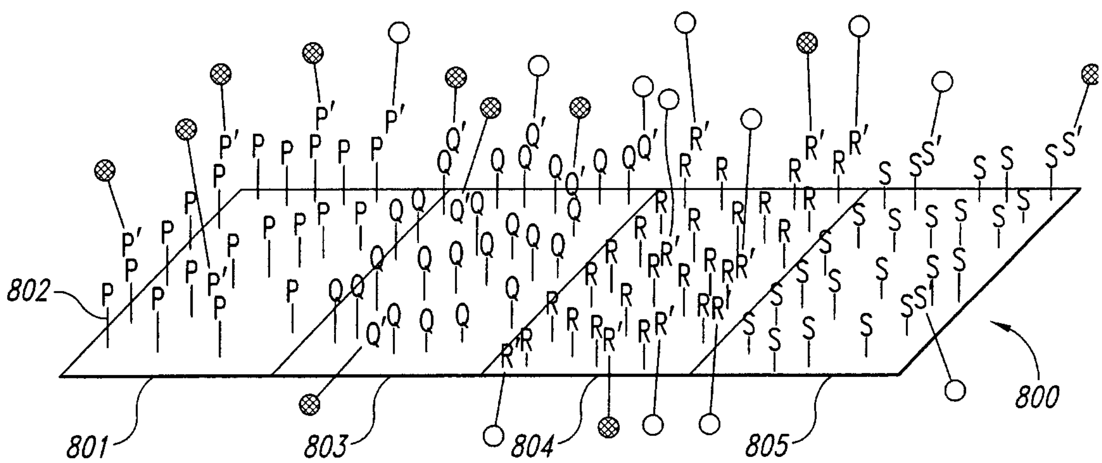


Fig. 8C

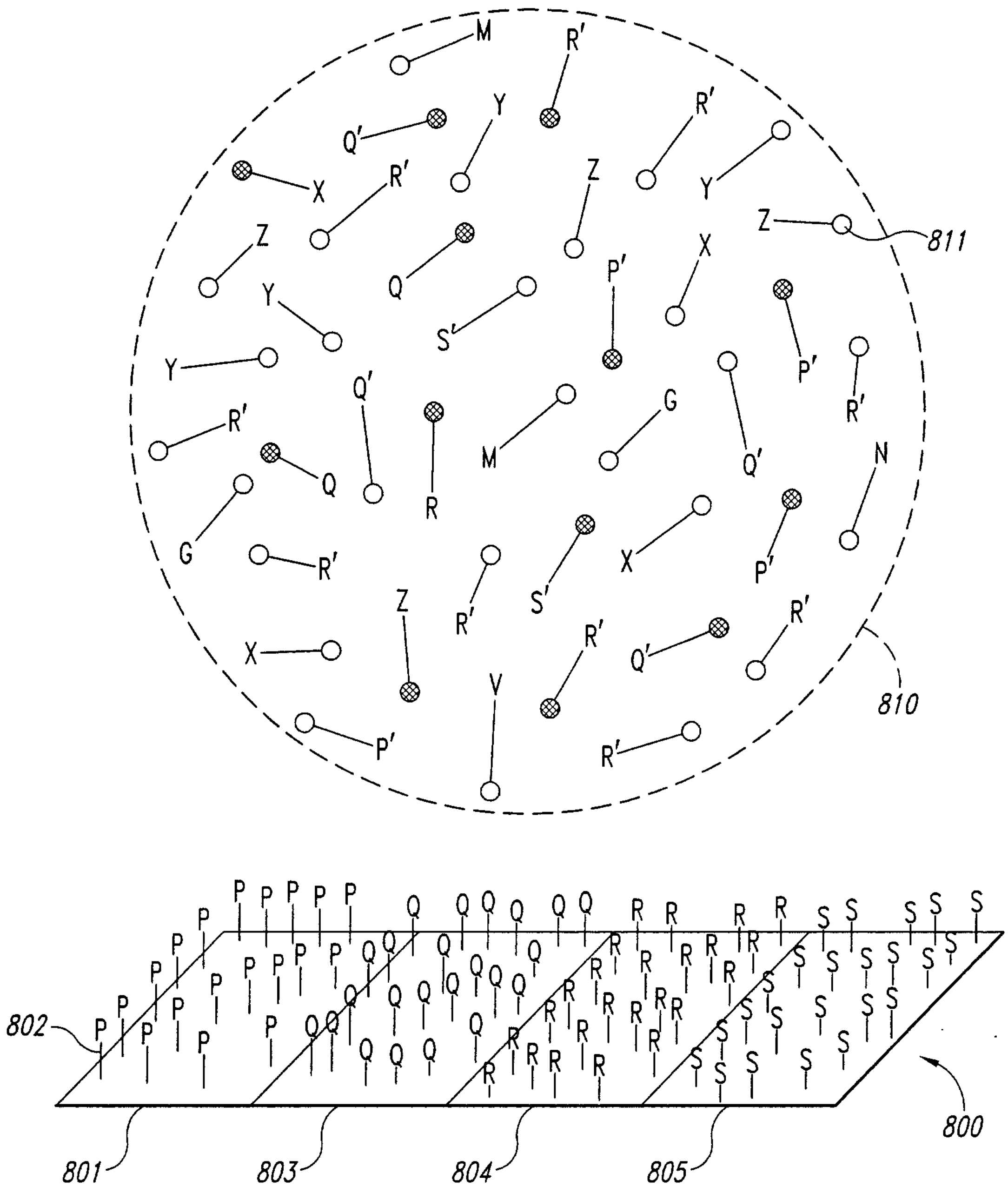


*Fig. 8B*

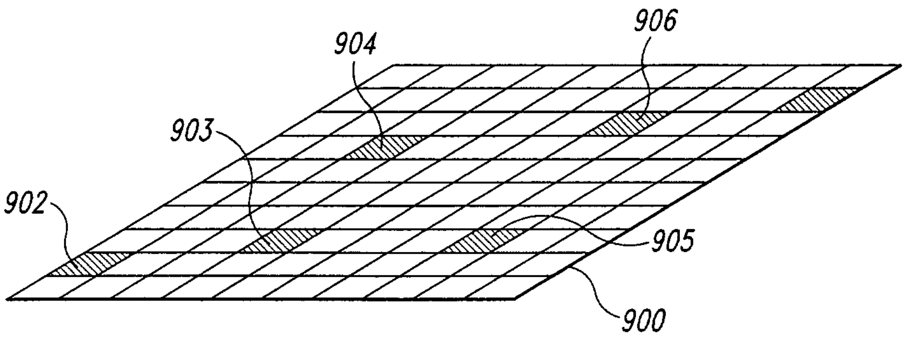


*Fig. 9*

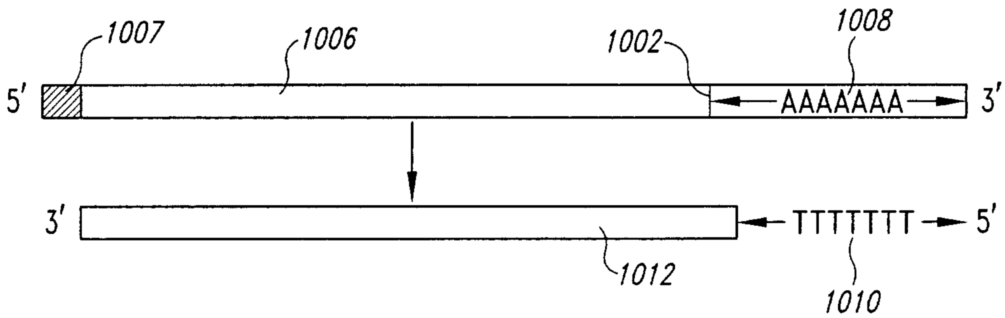


*Fig. 10*

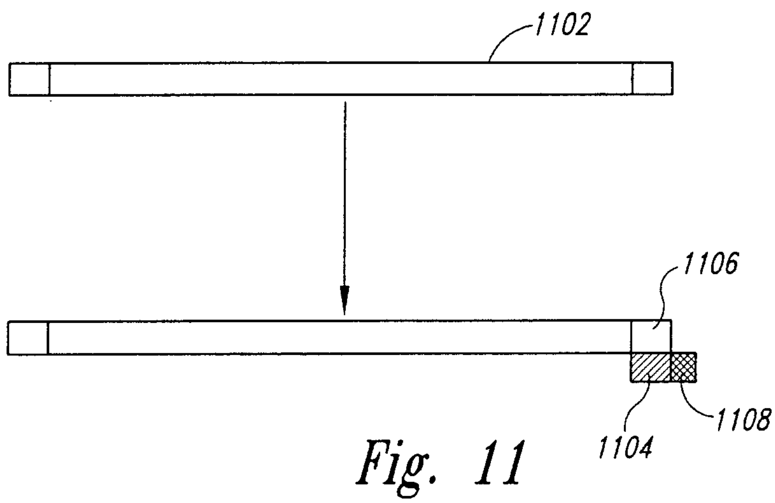


*Fig. 11*

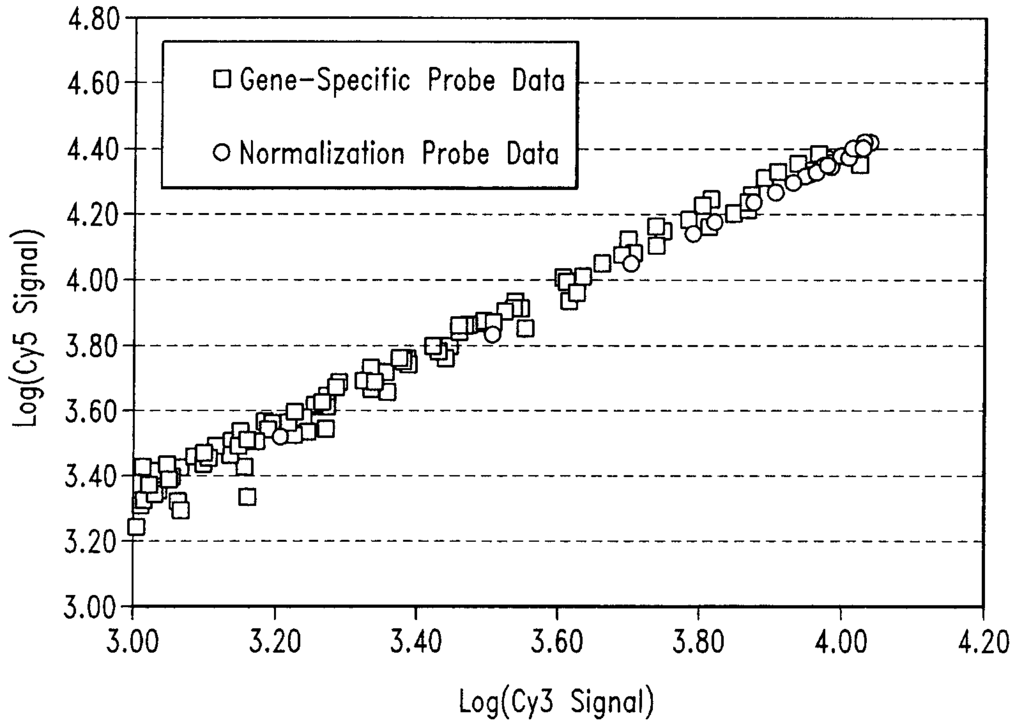
Gene-Specific Probe Data
Normalization Probe Data
4.80
4.60
4.40
4.20
4.00
3.80
3.60
3.40
3.20
3.00
Log(Cy5 Signal)
3.00
3.20
3.40
3.60
3.80
4.00
4.20
Log(Cy3 Signal)

*Fig. 12*